# EUROPÄISCHE PATENTSCHRIFT

(11) **EP 2 014 331 B1**
(45) Veröffentlichungstag und Bekanntmachung des Hinweises auf die Patenterteilung: **10.03.2010**
(21) Anmeldenummer: 08012342.5
(22) Anmeldetag: 09.07.2008
(51) Int. Cl.: A61M 37/00, A61B 17/00, A61B 17/88

(54) **Vorrichtung zur intrakorporalen Applikation medizinischer Hilfsmittel**
Device for intracorporal application of medical aids
Dispositif d'application intracorporelle des dispositifs médicaux

(30) Priorität: 12.07.2007 DE 102007032482
(43) Veröffentlichungstag der Anmeldung: 14.01.2009
(73) Patentinhaber: Karl Storz GmbH & Co. KG, 78532 Tuttlingen (DE)
(72) Erfinder: Kohl, Prof.Dr. Thomas, 53127 Bonn (DE); Blocher, Martin, 78532 Tuttlingen (DE)
(74) Vertreter: Hofmeister, Frank

(56) Entgegenhaltungen:
- EP-A- 0 581 036
- EP-A- 0 625 334
- WO-A-2007/056297
- US-A- 5 263 969
- US-A- 5 464 403
- US-A- 5 814 058

## Beschreibung

Die Erfindung betrifft eine_Vorrichtung zur intrakorporalen Applikation medizinischer Hilfsmittel, insbesondere Plastiken und Wundauflagen, mit einem hohlen Applikationsrohr zum Einbringen des medizinischen Hilfsmittels in das Operationsgebiet.

In verschiedenen Bereichen der Chirurgie, insbesondere der endoskopischen Chirurgie, ist es erforderlich medizinische Hilfsmittel, wie beispielsweise Wundauflagen oder Plastiken, in das Operationsgebiet einzubringen und vor Ort unter beengten Verhältnissen zu applizieren.

So ist es beispielsweise bekannt bei Hernien-Operationen (Leistenbruch-OP) die zu verschließende Faszienlücke mittels eines netzartigen Gewebes zu überbrücken, das über eine Trokarhülse in das Operationsgebiet eingebracht wird. Hierzu wird das Gewebenetz von Hand zusammengerollt oder gefaltet und in die Trokarhülse gesteckt und mittels einer Fasszange zum Operationsgebiet geschoben. Nachteilig an dieser beschriebenen Vorgehensweise ist neben dem großen manuellen Aufwand für das Rollen oder Falten und anschließende Einbringen des solchermaßen verkleinerten Gewebes in die Trokarhülse die Gefahr, dass das Gewebe beim Einschieben in die Trokarhülse beschädigt wird.

EP 0 625 334 A1, US 5 464 403, US 5 814 058, US 5 263 969, EP 0 581 036 A1 und WO 2007/056 297 A2 offenbaren Applikatoren mit verschiebbaren Wickeleinsätzen ohne Raststufen.

Davon ausgehend liegt der Erfindung die **Aufgabe** zugrunde, eine Vorrichtung zur intrakorporalen Applikation medizinischer Hilfsmittel, zu schaffen, die bei einfacher Handhabung eine sichere Applikation des medizinischen Hilfsmittels im Operationsgebiet gewährleistet.

Die L ö s u n g dieser Aufgabenstellung wird in Anspruch 1 dargestellt.

Durch die erfindungsgemäße Verwendung des in das Applikationsrohr einsetzbaren Wickeleinsatzes ist es erstmalig möglich, auf ein manuelles Rollen oder Falten des zu applizierenden medizinischen Hilfsmittels zu verzichten. Der Wickeleinsatz mitsamt dem auf den Wickeleinsatz aufgewickelten medizinischen Hilfsmittel ist überdies in Längsrichtung des Applikationsrohres verschiebbar, um das medizinische Hilfsmittel zum Operationsgebiet hin zu verschieben.

Mit einer praktischen Ausführungsform der Erfindung wird vorgeschlagen, dass der Wickeleinsatz und das Applikationsrohr im ineinandergesetzten Zustand um die Instrumentenlängsachse gegeneinander verdrehbar sind. Diese gegenseitige Verdrehbarkeit der Bauteile erleichtert das Auf- und Abwickeln des medizinischen Hilfsmittels auf den Wickeleinsatz bzw. vom Wickeleinsatz herunter.

Zum Festlegen des medizinischen Hilfsmittels am Wickeleinsatz wird mit einer ersten Ausführungsform der Erfindung vorgeschlagen, dass das medizinische Hilfsmittel am Wickeleinsatz festlegbar ist, beispielsweise durch Ausbildung eines Schlitzes im Wickeleinsatz zur Aufnahme eines Endes des medizinischen Hilfsmittels.

Gemäß einer alternativen Ausgestaltungsform erfolgt das Festlegen des medizinischen Hilfsmittels am Wickeleinsatz über einen im Wickeleinsatz ausgebildeten durchgehenden Schlitz, in den das medizinische Hilfsmittel einsetzbar ist.

Gemäß einer praktischen Ausführungsform der Erfindung wird vorgeschlagen, dass der Wickeleinsatz zumindest im das medizinische Hilfsmittel aufnehmenden Bereich als in das Applikationsrohr einsetzbarer Drahtstift, vorzugsweise aus einem Ni-Ti-Werkstoff, ausgebildet ist.

Um das Handhaben der erfindungsgemäßen Vorrichtung zu erleichtern, ist der Wickeleinsatz in vorgegebenen Raststufen in axialer Richtung in das Applikationsrohr einsetzbar. Diese Raststufen stellen vorgegebene Einstecktiefen des Wickeleinsatzes in das Applikationsrohr, beispielsweise eine Einführ- und eine Applikationsstellung, dar und ermöglichen so ein sicheres und gleichmäßiges Bedienen der Vorrichtung.

Weiterhin wird mit der Erfindung vorgeschlagen, dass im Applikationsrohr mindestens eine in Axialrichtung verlaufende Öffnung zum Einführen des medizinischen Hilfsmittels ausgebildet ist. Diese fensterartige Öffnung im Applikationsrohr ermöglicht das Festlegen des medizinischen Hilfsmittels am Wickeleinsatz bei in das Applikationsrohr eingesetztem Wickeleinsatz. Hierzu wird das medizinische Hilfsmittel durch die Öffnung in das Innere des Applikationsrohres geführt und beispielsweise mit einem Ende im Schlitz des Wickeleinsatzes festgelegt. Durch nachfolgendes Verdrehen des Wickeleinsatzes und/oder des Applikationsrohres wird das medizinische Hilfsmittel in das Applikationsrohr eingezogen und um den Wickeleinsatz gewickelt.

Gemäß einer praktischen Ausführungsform der Erfindung sind im Applikationsrohr zwei einander in etwa gegenüberliegende, in Axialrichtung verlaufende Öffnungen ausgebildet. Insbesondere bei großen respektive langen medizinischen Hilfsmitteln ist diese Ausgestaltungsform vorteilhaft, da es ein schnelleres Aufwickeln des medizinischen Hilfsmittels auf den Wickeleinsatz ermöglicht. Hierzu wird das medizinische Hilfsmittel durch eine Öffnung in das Innere des Applikationsrohres geführt und durch den durchgehenden Schlitz des Wickeleinsatzes hindurchgezogen, bis das medizinische Hilfsmittel aus der gegenüberliegenden Öffnung des Applikationsrohres wieder aus dem Applikationsrohr austritt. Durch nachfolgendes Verdrehen des Wickeleinsatzes und/oder des Applikationsrohres wird das medizinische Hilfsmittel in das Applikationsrohr eingezogen und doppelgängig um den Wickeleinsatz gewickelt.

Das Bedienen des Applikationsrohres und des Wickeleinsatzes, insbesondere beim gegenseitigen Verdrehen der Bauteile, kann erfindungsgemäß dadurch erleichtert werden, dass am proximalen Ende des Applikationsrohres und/oder des Wickeleinsatzes ein Handgriff angeordnet ist.

Schließlich wird mit der Erfindung vorgeschlagen, dass das medizinische Hilfsmittel eine Netzplastik für eine Hernien-OP oder dergleichen ist. Bei der Hernien-OP werden die aus körpereigenem oder körperfremdem Gewebe bestehenden Netzplastiken oder Netzimplantate verwendet, um die entsprechende Faszienlücke zu überbrücken.

Weitere Merkmale und Vorteile der Erfindung ergeben sich anhand der zugehörigen Zeichnung, in der zwei Ausführungsbeispiele einer erfindungsgemäßen Vorrichtung zur intrakorporalen Applikation medizinischer Hilfsmittel nur beispielhaft dargestellt sind, ohne die Erfindung auf diese Ausführungsbeispiele zu beschränken. In der Zeichnung zeigt:
- Fig. 1: eine Seitenansicht einer erfindungsgemäßen Vorrichtung zur intrakorporalen Applikation medizinischer Hilfsmittel;
- Fig. 2: einen Längsschnitt entlang der Linie II-II gemäß Fig. 1;
- Fig. 3: einen vergrößerten Querschnitt entlang der Linie III-III gemäß Fig. 1;
- Fig. 4: eine Fig. 3 entsprechende Schnittansicht, jedoch eine zweite erfindungsgemäße Ausführungsform darstellend;
- Fig. 5: eine schematische Darstellung der Aufwicklung des medizinischen Hilfsmittels auf einen Wickeleinsatz gemäß Fig. 3 und
- Fig. 6: eine schematische Darstellung der Aufwicklung des medizinischen Hilfsmittels auf einen Wickeleinsatz gemäß Fig. 4.

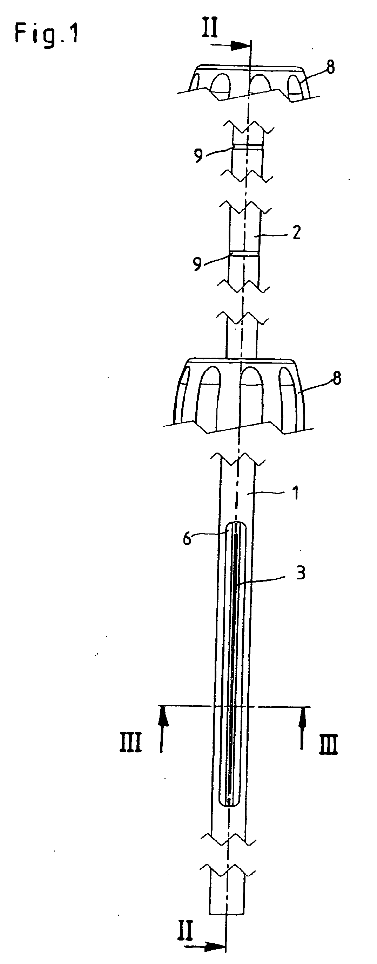

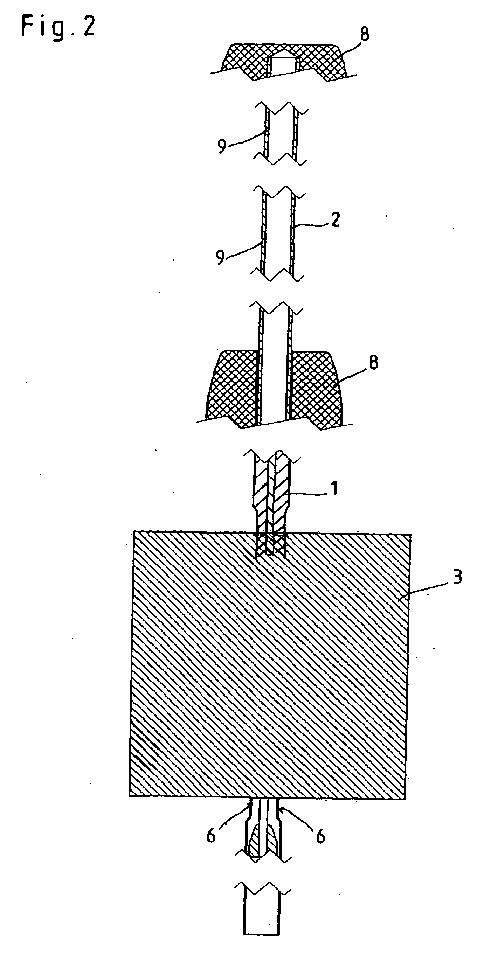

Die in den Abbildungen Fig. 1 und 2 dargestellte Vorrichtung zur intrakorporalen Applikation medizinischer Hilfsmittel besteht im Wesentlichen aus einem hohlen äußeren Applikationsrohr 1 und einem in das Applikationsrohr 1 einsetzbaren und in Längsrichtung des Applikationsrohres 1 verschiebbaren Wickeleinsatz 2.

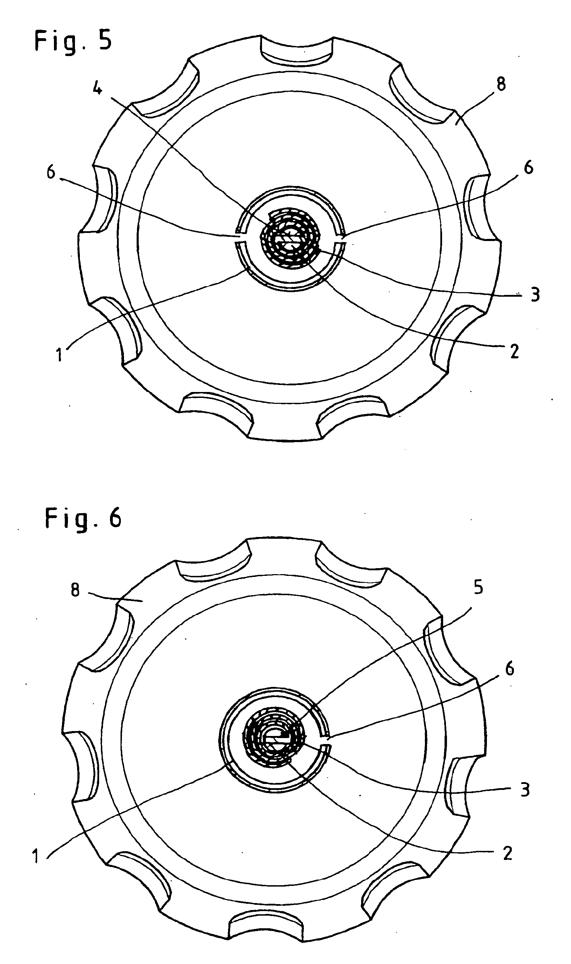

Wie insbesondere aus Fig. 5 und 6 ersichtlich, dient der Wickeleinsatz 2 zur haltenden Aufnahme eines medizinischen Hilfsmittels 3, das über das Applikationsrohr 1 in das Operationsgebiet eingebracht wird. Hierzu wird das medizinische Hilfsmittel 3 am Wickeleinsatz 2 festgelegt und auf den Wickeleinsatz 2 aufgewickelt, bevor das medizinische Hilfsmittel 3 nachfolgend in diesem auf den Wickeleinsatz 2 aufgewickelten Zustand durch das Applikationsrohr 1 hindurch dem Operationsgebiet zugeführt wird.

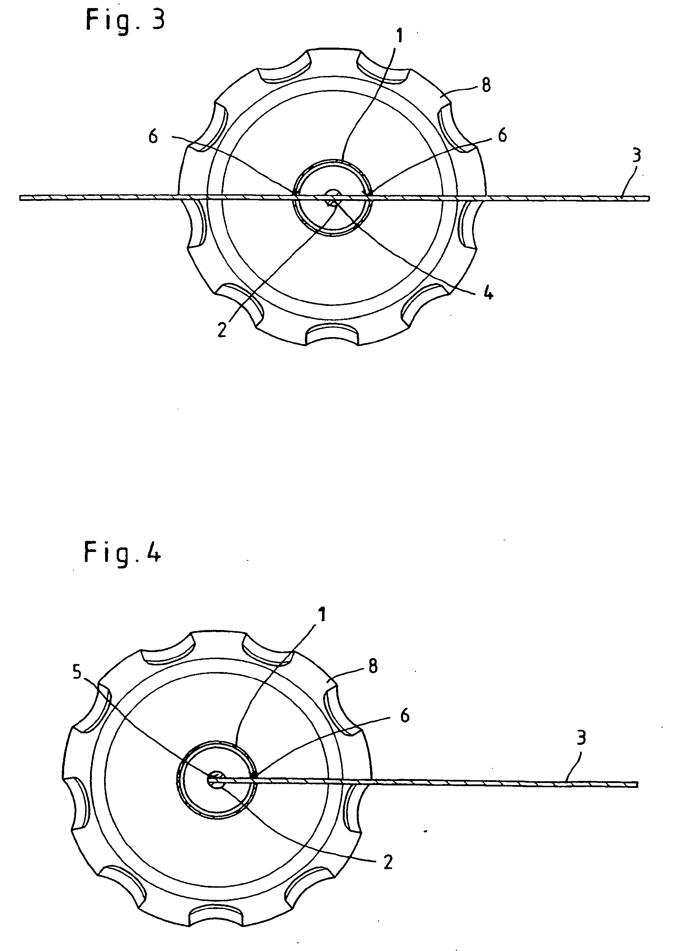

Das Festlegen des medizinischen Hilfsmittels 3 am Wickeleinsatz 2 erfolgt bei der in Fig. 3 dargestellten ersten Ausführungsform über einen im Wickeleinsatz 2 ausgebildeten durchgehenden Schlitz 4, durch den das medizinische Hilfsmittel 3 hindurchgeschoben wird, bis das medizinische Hilfsmittel 3 beidseitig, vorzugsweise mit gleicher Länge, aus dem Schlitz 4 herausragt. Durch Drehen des Wickeleinsatzes 2 lässt sich nunmehr das medizinische Hilfsmittel 3 doppelgängig auf den Wickeleinsatz 2 aufwickeln, wie dies schematisch in Fig. 5 dargestellt ist.

Gemäß der in Fig. 4 dargestellten zweiten Ausführungsform erfolgt das Festlegen des medizinischen Hilfsmittels 3 am Wickeleinsatz 2 derart, dass im Wickeleinsatz 2 ein Schlitz 5 ausgebildet ist, in den ein Ende des medizinischen Hilfsmittels 3 einsteckbar ist. Durch Drehen des Wickeleinsatzes 2 lässt sich nunmehr das medizinische Hilfsmittel 3 eingängig auf den Wickeleinsatz 2 aufwickeln, wie dies schematisch in Fig. 6 dargestellt ist.

Bei den medizinischen Hilfsmitteln 3 kann es sich beispielsweise um Wundauflagen oder Plastiken handeln, die in zusammengelegter Form in das Operationsgebiet eingebracht werden und erst an Ort und Stelle am Operationsgebiet ausgebreitet und auf den entsprechenden Anwendungsort aufgebracht werden.

Ein Anwendungsgebiet für diese Vorrichtung stellen beispielsweise Hernien-Operationen dar, bei denen das medizinische Hilfsmittel als aus körpereigenem oder körperfremdem Gewebe bestehende Netzplastik oder Netzimplantatausgebildet ist, um die entsprechende Faszienlücke zu überbrücken.

In Betracht kommen aber prinzipiell alle chirurgischen Anwendungsbereiche, bei denen eine Wundstelle oder Öffnung mittels eines flächigen medizinischen Hilfsmittels 3 überbrückt und/oder verschlossen werden soll, wie beispielsweise bei der Chirurgie im Bauchraum bei Magen- oder Darmdurchbrüchen oder in der Fetoskopie zum Verschließen der Spina bifida. Aber auch die intrakorporale Versorgung von Wunden, beispielsweise fetaler Kopfwunden, mit Pflastern und dergleichen flächigen Wundauflagen ist mit dieser Vorrichtung möglich.

Neben der Möglichkeit, das medizinische Hilfsmittel 3 außerhalb des Applikationsrohres 1 auf den Wickeleinsatz 2 aufzuwickeln und erst nachfolgend den mit dem medizinischen Hilfsmittel 3 umwickelten Wickeleinsatz 2 in das Applikationsrohr 1 einzuführen, besteht die bevorzugte und in den Abbildungen Fig. 1 bis Fig. 6 dargestellte Möglichkeit, das medizinische Hilfsmittel 3 erst im in das Applikationsrohr 1 eingesetzten Zustand des Wickeleinsatzes 2 am Wickeleinsatz 2 festzulegen und auf den Wickeleinsatz 2 aufzuwickeln.

Hierzu ist in der Mantelfläche des Applikationsrohres 1 mindestens eine in Axialrichtung des Applikationsrohres 1 verlaufende fensterartige Öffnung 6 ausgebildet, durch die das medizinische Hilfsmittel 3 zum Festelegen am Wickeleinsatz 2 in das Applikationsrohr 1 einführbar ist, wie dies in Fig. 1 dargestellt ist. Bei der in Fig. 1 dargestellten Ausführungsform des Applikationsrohres 1 ist die fensterartige Öffnung 6 als Spalt 6 ausgebildet, jedoch sind auch andere Ausgestaltungsformen möglich, die es erlauben, das medizinische Hilfsmittel 3 zum Festelegen am Wickeleinsatz 2 in das Applikationsrohr 1 einzuführen.

Für die in Fig. 4 und 6 dargestellte Ausführungsform, bei der das medizinische Hilfsmittel 3 eingängig um den Wickeleinsatz 2 gewickelt wird, ist es ausreichend, im Applikationsrohr 1 nur einen Spalt 6 auszubilden. Die in den Abbildungen Fig. 4 und 6 dargestellten Ausführungsformen unterscheiden sich jedoch hinsichtlich der im Wickeleinsatz 2 ausgebildeten Schlitze 4 und 5. Während gemäß Fig. 4 im Wickeleinsatz 2 nur ein Schlitz 5 ausgebildet ist, in den ein Ende des medizinischen Hilfsmittels 3 einsteckbar ist, ist bei der Ausführungsform gemäß Fig. 6 im Wickeleinsatz 2 ein durchgehender Schlitz 4 ausgebildet, durch den das medizinische Hilfsmittel 3 hindurchsteckbar ist. Diese Ausgestaltungsform bietet dem am Wickeleinsatz 2 festgelegten medizinischen Hilfsmittel 3 einen besseren Halt als die Verwendung des nicht durchgehenden Schlitzes 5.

Die in Fig. 2 und 5 dargestellte Ausführungsform mit dem im Wickeleinsatz 2 ausgebildeten durchgehenden Schlitz 5 und dem doppelgängig aufgewickelten medizinischen Hilfsmittel 3 erfordert dahingegen zwei Spalte 6 im Applikationsrohr 1, damit das medizinische Hilfsmittel 3 nach dem Festlegen im Schlitz 5 des Wickeleinsatzes 2 auch beidseitig aus dem Applikationsrohr 1 herausragen kann.

Das Aufwickeln des solchermaßen am Wickeleinsatz 2 festgelegten medizinischen Hilfsmittels 3 auf den Wickeleinsatz 2 erfolgt nachfolgend durch Drehen des Wickeleinsatzes 2 und/oder des Applikationsrohres 1 um die Instrumentenlängsachse 7, wobei für den Fall, dass beide Bauteile 1 und 2 gedreht werden, das Applikationsrohr 1 und der Wickeleinsatz 2 gegenläufig gedreht werden müssen. Durch diese Drehung mindestens eines Bauteils 1 oder 2 relativ zu dem anderen Bauteil 2 oder 1 wird das am Wickeleinsatz 2 festgelegte medizinische Hilfsmittel 3 in das Applikationsrohr 1 hineingezogen und lagenweise um den Wickeleinsatz 2 gewickelt.

Um sicherzustellen, dass der Wickeleinsatz 2 auch noch mit dem darauf aufgewickelten medizinischen Hilfsmittel 3 im Applikationsrohr 1 axialverschiebbar ist, ist der Wickeleinsatz 2 zumindest im das medizinische Hilfsmittel 3 aufnehmenden Bereich als den Durchmesser des Wickeleinsatzes 2 reduzierender Drahtstift ausgebildet, wie dies der Abbildung Fig. 2 zu entnehmen ist. Abweichend von der dargestellten Form des Drahtstiftes mit kreisförmigem Querschnitt sind selbstverständlich auch andere Querschnittsformen ausführbar.

In den übrigen Teilbereichen ist der Wickeleinsatz 2 vorteilhafterweise rohrförmig ausgebildet, wobei der Außendurchmesser des Wickeleinsatzes 2 im Wesentlichen dem Innendurchmesser des Applikationsrohres 1 entspricht, um eine gute und verkantungsfreie Führung des Wickeleinsatzes 2 im Applikationsrohr 1 zu gewährleisten.

Das Bedienen des Applikationsrohres 1 und des Wickeleinsatzes 2, insbesondere das Verdrehen der Bauteile 1 und 2, wird bei der dargestellten Ausführungsform dadurch erleichtert, dass am proximalen Ende sowohl des Applikationsrohres 1 als auch des Wickeleinsatzes 2 jeweils ein möglichst ergonomisch ausgestalteter Handgriff 8 angeordnet ist.

Das Zusammensetzen und Bedienen der wie voranstehend beschrieben aufgebauten Vorrichtung zur intrakorporalen Applikation medizinischer Hilfsmittel geschieht wie folgt:
Im ersten Arbeitsschritt wird der Wickeleinsatz 2 mit dem distalen Ende voran vom proximalen Ende her in das hohle Applikationsrohr 1 gesteckt, bis der Wickeleinsatz 2 die in Fig. 1 dargestellte Position einnimmt, in der der im Wickeleinsatz 2 ausgebildete durchgehende Schlitz 4, oder auch nur der einseitige Schlitz 5, im Bereich des mindestens einen fensterartigen Spalts 6 des Applikationsrohres 1 angeordnet ist.

Um für diesen ersten Arbeitsschritt und auch die nachfolgenden Arbeitsschritte immer die richtige Einstecktiefe des Wickeleinsatzes 2 in das Applikationsrohr 1 einzuhalten, sind, wie aus Fig. 1 ersichtlich, bei der dargestellten Ausführungsform verschiedene Raststufen 9 am Wickeleinsatz 2 ausgebildet, wobei jede Raststufe 9 einer bestimmten Einstecktiefe des Wickeleinsatzes 2 in das Applikationsrohr 1 entspricht und einen entsprechenden Arbeitsschritt markiert.

Nach dem Einführen des Wickeleinsatzes 2 in das Applikationsrohr 1 bis zu der zuvor beschriebenen ersten Raststufe wird nachfolgend das medizinische Hilfsmittel 3 am Wickeleinsatz 2 festgelegt, beispielsweise durch Hindurchstecken durch den durchgehenden Schlitz 4, wie dies in Fig. 2 dargestellt ist. Nachfolgend werden der Wickeleinsatz 2 und/oder das Applikationsrohr 1 relativ zueinander um die Instrumentenlängsachse 7 verdreht, wodurch das medizinische Hilfsmittel in das Applikationsrohr 1 hineingezogen wird und sich auf den Wickeleinsatz 2 aufwickelt.

Im nächsten Schritt wird der mit dem aufgewickelten medizinischen Hilfsmittel 3 versehene Wickeleinsatz 2 in distaler Richtung weiter in das Applikationsrohr 1 hineingeschoben, bis eine zweite Raststufe 9 erreicht ist, in der das medizinische Hilfsmittel 3 im Inneren des Applikationsrohres 1 angeordnet ist und nicht wieder über den mindestens einen Spalt 6 des Applikationsrohres 1 von dem Wickeleinsatz 2 abgewickelt werden kann.

In dieser Arbeitsstellung wird das Applikationsrohr 1 mitsamt dem eingesteckten Wickeleinsatz 2, beispielsweise über eine Trokarhülse in das Operationsgebiet eingeführt. Im Operationsgebiet wird der Wickeleinsatz 2 nunmehr bis zur dritten Raststufe 9 in das Applikationsrohr 2 hineingedrückt, in der der mit dem zu applizierenden medizinischen Hilfsmittel 3 umwickelte Wickeleinsatz 2 distalseitig wieder aus dem Applikationsrohr 1 heraustritt. Nun kann das medizinische Hilfsmittel 3 beispielsweise mittels einer Fasszange erfasst werden. Durch Drehen des Wickeleinsatzes 2 lässt sich das medizinische Hilfsmittel 3 nunmehr einfach und sicher an Ort und Stelle vom Wickeleinsatz 2 abwickeln und auf die zu versorgende Operationsstelle aufbringen.

Eine solchermaßen ausgebildete Vorrichtung zur intrakorporalen Applikation medizinischer Hilfsmittel 3 zeichnet sich dadurch aus, dass sie bei einfachem Aufbau und einfacher Handhabung einen sicheren und schonenden Transport des zu applizierenden medizinischen Hilfsmittels 3 hin zur Operationsstelle gewährleistet.

### Bezugszeichenliste

- 1: Applikationsrohr
- 2: Wickeleinsatz
- 3: medizinisches Hilfsmittel
- 4: Schlitz (durchgehend)
- 5: Schlitz
- 6: Öffnung / Spalt
- 7: Instrumentenlängsachse
- 8: Handgriff
- 9: Raststufe

## Patentansprüche

1. Vorrichtung zur intrakorporalen Applikation medizinischer Hilfsmittel, insbesondere Plastiken und Wundauflagen, mit einem hohlen Applikationsrohr (1) zum Einbringen des medizinischen Hilfsmittels (3) in das Operationsgebiet sowie mit einem in das hohle Applikationsrohr (1) einsetzbaren und in Längsrichtung des Applikationsrohres (1) verschiebbaren Wickeleinsatz (2), auf den das zu applizierende medizinische Hilfsmittel (3) aufwickelbar ist,
**dadurch gekennzeichnet,**
**dass** der Wickeleinsatz (2) in vorgegebenen Raststufen (9) in axialer Richtung in das Applikationsrohr (1) einsetzbar ist.

2. Vorrichtung nach Anspruch 1, **dadurch gekennzeichnet, dass** der Wickeleinsatz (2) und das Applikationsrohr (1) im ineinandergesetzten Zustand um die Instrumentenlängsachse (7) gegeneinander verdrehbar sind.

3. Vorrichtung nach Anspruch 1 oder 2, **dadurch gekennzeichnet, dass** das medizinische Hilfsmittel (3) am Wickeleinsatz (2) festlegbar ist.

4. Vorrichtung nach Anspruch 3, **dadurch gekennzeichnet, dass** im Wickeleinsatz (2) ein Schlitz (5) zur Aufnahme eines Endes des medizinischen Hilfsmittels (3) ausgebildet ist.

5. Vorrichtung nach Anspruch 3, **dadurch gekennzeichnet, dass** im Wickeleinsatz (2) ein durchgehender Schlitz (4) zur Aufnahme des medizinischen Hilfsmittels (3) ausgebildet ist.

6. Vorrichtung nach einem der Ansprüche 1 bis 5, **dadurch gekennzeichnet, dass** der Wickeleinsatz (2) zumindest im das medizinische Hilfsmittel (3) aufnehmenden Bereich als in das Applikationsrohr (1) einsetzbarer Drahtstift ausgebildet ist.

7. Vorrichtung nach einem der Ansprüche 1 bis 6, **dadurch gekennzeichnet, dass** im Applikationsrohr (1) mindestens eine in Axialrichtung verlaufende Öffnung (6) zum Einführen des medizinischen Hilfsmittels (3) ausgebildet ist.

8. Vorrichtung nach Anspruch 7, **dadurch gekennzeichnet, dass** im Applikationsrohr (1) zwei einander in etwa gegenüberliegende, in Axialrichtung verlaufende Öffnungen (6) ausgebildet sind.

9. Vorrichtung nach einem der Ansprüche 1 bis 8, **dadurch gekennzeichnet, dass** am proximalen Ende des Applikationsrohres (1) und/oder des Wickeleinsatzes (2) ein Handgriff (8) angeordnet ist.

10. Vorrichtung nach einem der Ansprüche 1 bis 9, **dadurch gekennzeichnet, dass** das medizinische Hilfsmittel (3) eine Netzplastik für eine Hernien-OP oder dergleichen ist.

## Claims

1. Device for intracorporeal application of medical aids, in particular grafts and wound dressings, with a hollow application tube (1) for inserting the medical aid (3) into the operating site, and with a winding insert (2) which can be inserted into the hollow application tube (1) and can be moved in the longitudinal direction of the application tube (1) and onto which the medical aid (3) to be applied can be wound, **characterized in that** the winding insert (2) can be inserted into the application tube (1) in predetermined locking steps (9) in the axial direction.

2. Device according to Claim 1, **characterized in that** the winding insert (2) and the application tube (1), when inserted one inside the other, can be rotated with respect to each other about the longitudinal axis (7) of the instrument.

3. Device according to Claim 1 or 2, **characterized in that** the medical aid (3) can be secured on the winding insert (2).

4. Device according to Claim 3, **characterized in that** a slit (5) for receiving one end of the medical aid (3) is formed in the winding insert (2).

5. Device according to Claim 3, **characterized in that** a continuous slit (4) for receiving the medical aid (3) is formed in the winding insert (2).

6. Device according to one of Claims 1 to 5, **characterized in that** the winding insert (2), at least in the area receiving the medical aid (3), is designed as a wire pin that can be inserted into the application tube (1).

7. Device according to one of Claims 1 to 6, **characterized in that** the application tube (1) has at least one opening (6) extending in the axial direction and serving for insertion of the medical aid (3).

8. Device according to Claim 7, **characterized in that** the application tube (1) has two openings (6) located approximately opposite each other and extending in the axial direction.

9. Device according to one of Claims 1 to 8, **characterized in that** a handle (8) is arranged on the proximal end of the application tube (1) and/or of the winding insert (2).

10. Device according to one of Claims 1 to 9, **characterized in that** the medical aid (3) is a mesh graft for a hernia operation or the like.

## Revendications

1. Appareillage pour l'application intracorporelle de dispositifs médicaux, notamment de greffes plastiques et de pansements, comprenant un tube d'application creux (1) destiné à introduire le dispositif médical (3) dans la région opératoire, ainsi qu'un module d'enroulement (2) qui peut être inséré dans le tube d'application creux (1) et peut coulisser dans la direction longitudinale du tube d'application (1), et sur lequel peut être enroulé le dispositif médical (3) à appliquer, **caractérisé en ce que** le module d'enroulement (2) peut être inséré dans le tube d'application (1) selon des gradins d'encliquetage (9) prédéfinis dans la direction axiale.

2. Appareillage selon la revendication 1, **caractérisé en ce que** le module d'enroulement (2) et le tube d'application (1) peuvent, dans l'état où ils sont insérés l'un dans l'autre, être tournés l'un par rapport à l'autre autour de l'axe longitudinal (7) de l'instrument.

3. Appareillage selon la revendication 1 ou la revendication 2, **caractérisé en ce que** le dispositif médical (3) peut être fixé au module d'enroulement (2).

4. Appareillage selon la revendication 3, **caractérisé en ce que** dans le module d'enroulement (2) est réalisée une fente (5) destinée à recevoir une extrémité du dispositif médical (3).

5. Appareillage selon la revendication 3, **caractérisé en ce que** dans le module d'enroulement (2) est réalisée une fente traversante (4) destinée à recevoir le dispositif médical (3).

6. Appareillage selon l'une des revendications 1 à 5, **caractérisé en ce que** le module d'enroulement (2) est réalisé, au moins dans la zone recevant le dispositif médical (3), en tant que broche filaire pouvant être insérée dans le tube d'application (1).

7. Appareillage selon l'une des revendications 1 à 6, **caractérisé en ce que** dans le tube d'application (1) est réalisée au moins une ouverture (6) s'étendant dans la direction axiale et destinée à l'introduction du dispositif médical (3).

8. Appareillage selon la revendication 7, **caractérisé en ce que** dans le tube d'application (1) sont réalisées deux ouvertures (6) sensiblement opposées l'une à l'autre et s'étendant dans la direction axiale.

9. Appareillage selon l'une des revendications 1 à 8, **caractérisé en ce qu'**à l'extrémité proximale du tube d'application (1) et/ou du module d'enroulement (2), est agencée une poignée (8).

10. Appareillage selon l'une des revendications 1 à 9, **caractérisé en ce que** le dispositif médical (3) est un filet prothétique en matière plastique pour une opération de hernie ou similaire.
